# EUROPEAN PATENT SPECIFICATION

(11) **EP 1 207 787 B1**
(45) Date of publication and mention of the grant of the patent: **09.01.2008**
(21) Application number: 00953302.7
(22) Date of filing: 14.08.2000
(51) Int. Cl.: A61B 6/00, A61N 5/10

(54) **PORTAL IMAGING DEVICE**
BILDERZEUGUNGSGERÄT
SYSTEME DE CENTRAGE

(30) Priority: 17.08.1999 GB 9919274
(43) Date of publication of application: 29.05.2002
(73) Proprietor: Elekta AB (publ), 103 93 Stockholm (SE)
(72) Inventor: COOKE, Robert, Stephen, Ewhurst, Surrey GU6 7QA (GB); FRYER, Christopher, John, Droitwich, Worcestershire WR9 0BA (GB); HARWOOD, William, Richard, 26 Orchard Way, Hassocks, West Sussex BN6 9UB (GB); PERKINS, Clifford, William, Crawley, West Sussex RH10 6NG (GB); STREAMER, Ralph, Peter, Horsham, West Sussex RH12 5RS (GB)
(74) Representative: Townsend, Victoria Jayne
(86) International application number: PCT/GB2000/003117
(87) International publication number: WO 2001/012066

(56) References cited:
- EP-A- 0 631 088
- EP-A- 0 919 186
- WO-A-96/03077
- DE-A- 19 640 048
- US-A- 4 995 068
- US-A- 5 631 943

## Description

This invention relates to radiation treatment apparatus, in particular portal imaging systems comprising a rotatable gantry, supported by a stand, a radiation emitting head coupled to the gantry and an imaging device for providing, in visual form, a representation of the radiation beam emitted from the head after it has passed through the object under treatment. In particular the invention relates to apparatus for mounting the imaging device on the gantry of the radiation treatment apparatus.

European Patent No. EP 0541717 identifies a problem with portal imaging devices, that being that in order for the lightweight boxes to cover a reasonable radiation field size, the construction of the detector enclosure has to be very bulky, this poses an inconvenience during patient set up and occupies space in the treatment room when not being used. Practical use of such devices has thus, historically been quite limited. That Patent goes on to describe an apparatus for mounting the imaging device to the gantry of a radiation treatment apparatus in which the imaging device is fixed to the end of a telescopically extendable holding means the holding means being arranged such that when not in use the majority of the holding apparatus and imaging device is retracted into the body of the gantry.

Whilst this arrangement provides a convenient means of storing the imaging device and associated mounting apparatus, the mounting apparatus is integral with the gantry of the radiation treatment apparatus and must therefore be built into the apparatus during manufacture. Additional disadvantages of this arrangement arise where parts of the mounting apparatus require repair maintenance or replacement. The arrangement also requires that space be found in the body of an already cumbersome piece of equipment to locate the collapsed mounting means.

Alternative mounting apparatus have been proposed which are surface mountable on the gantry of the radiation treatment apparatus. These arrangements comprise an arm for holding the imaging device which is collapsible about one or more pivots. The arm itself is pivotally mounted on the surface of the gantry of the treatment apparatus and the imaging device is pivotally connected to the distal end of the arm the arrangement being such that the entire assembly can be folded flat to sit flush against the surface of the gantry.

EP 0919186 (Picker International, Inc) discloses an apparatus for positioning an imaging device relative to the gantry of a radiation therapy apparatus. The apparatus comprises a C-arm having an imaging device secured to it by means of a cantilevered support bracket. The C-arm is secured to the gantry by a series of pivotally and telescopically linked arms. Whilst the arrangement permits positioning of the imaging device in a wide range of positions, the apparatus is cumbersome and potentially obstructive to persons using the radiation therapy apparatus.

In order to produce an accurate visual representation of the image it is essential that the imaging device is positioned and maintained in position accurately at a predetermined distance from the radiation emitting head. Thus, foldable mounting apparatus such as that described, is extremely difficult and expensive to engineer in practice and not always as accurate as may be desired.

It is therefore an object of the present invention to provide a mounting apparatus for mounting the imaging device on the gantry which alleviates some or all of the aforementioned disadvantages associated with the previously described mounting apparatus.

In accordance with the present invention there is provided apparatus for positioning an imaging device relative to the gantry of a radiation therapy apparatus as set out in claim 1.

Conveniently, the arm comprises two or more elongate elements arranged in slidable communication with each other. The slidable communication is provided by means of one or more linear bearings located between the elongate elements. The elongate elements may optionally be arranged to slide one inside another or alternatively side by side. To provide optimum stability, the arm is preferably pivotally mounted substantially about its centre of mass. Most preferably the arm is pivotally mounted substantially about the centre of mass of the arm and imaging device assembly.

The holder is slidably mounted to slide along the extendable arm. This arrangement provides for more compact retraction of the arm and image device assembly as well as more freedom in positioning the image device relative to the radiation head. The holder further comprises means for permitting linear motion of the imager device along an axis perpendicular to the longitudinal axis of the extendable arm. This arrangement provides a further degree of freedom in positioning the imaging device with respect to the radiation emitting head and when provided along with a slidably mounted holder provides for the imaging device to be easily locatable about a relatively large area.

As a further option, the apparatus may be provided with means for moving the image device radially along the surface of the gantry, toward and away from its centre point. Such means may comprise, for example, a slider on the surface of the gantry or a pivot and linkage system connecting the components of the apparatus.

Preferably the holder is detachable from the imaging device, permitting the imaging device to be removed for storage or replacement. Preferably, the holder has means for locking the position of the imaging device when the device is located within the holder.

In order to permit a further degree of freedom in positioning the imaging device, the holder may optionally comprise a rotating means for rotating the imaging device about an axis parallel to the longitudinal axis of the extending arm.

Preferably the apparatus is provided with counterbalancing means such that the arm and/or the arm and image device assembly can be held under gravity in any given angular position relative to the surface of the gantry.

Optionally, the apparatus may be activated by mechanical means, in particular, where the apparatus is provided with counterbalancing means, the sliding and rotational movements may be actuated by a simple lower power means.

Thus it can be seen that the invention provides a mechanically simple and inexpensive means of positioning an imaging device relative to the gantry of a radiation therapy apparatus. As the arrangement is surface mountable it can be fixed to existing equipment and can easily be maintained repaired or replaced. Apparatus according to the invention will operate at any given positional rotation of the gantry. Thus, two or more apparatus according to the present invention may be provided on any single given gantry of the radiation therapy apparatus. For example, two such apparatus may be disposed at two positions about the gantry of a radiation therapy apparatus a first position being commensurate with a megavolt measurement of the radiation image and the second position being commensurate with a kilovolt measurement of the radiation image. In this arrangement where the holder of the apparatus is provided to be detachable from the imaging device, a single imaging device may be transferred between the two apparatus according to the present invention to obtain both megavolt and kilovolt measurements.

A particular advantage of this arrangement is provided where the pivot about which the arm is mounted is offset from the end of the arm, this enables the arrangement to be self counter balancing when retracted and minimises any movement about the arm when extended. The inherent stability of this arrangement means that the forces to be overcome on extension and retraction are primarily frictional or inertial and can easily be overcome either manually or with simple electro-mechanical actuation devices.

It is also to be appreciated that the small framed lightweight arrangement is easy to manoeuvre around even when partially stowed.

The invention will now be further described by way of example with reference to the Figures, in which:-
Figure 1 shows an apparatus for positioning an imaging device relative to the gantry of radiation therapy apparatus substantially as described in European Patent EP 0541717;
Figure 2 shows a schematic of the surface mountable folding apparatus also described above;
Figure 3 shows an embodiment of the present invention in its fully extended position in both a perspective and side view;
Figure 4 shows the embodiment of Figure 3 in a partially retracted position;
Figure 5 shows the embodiment of Figure 3 with the arms and imaging device fully retracted;
Figure 6 shows how the fully retracted arm and imaging device rotate about a centre of mass to be stowed flat against the gantry surface;
Figure 7 illustrates the embodiment of Figure 3 in a fully stowed and retracted position in both a perspective and side view;
Figure 8 shows how the embodiment can operate at an alternative gantry rotation.

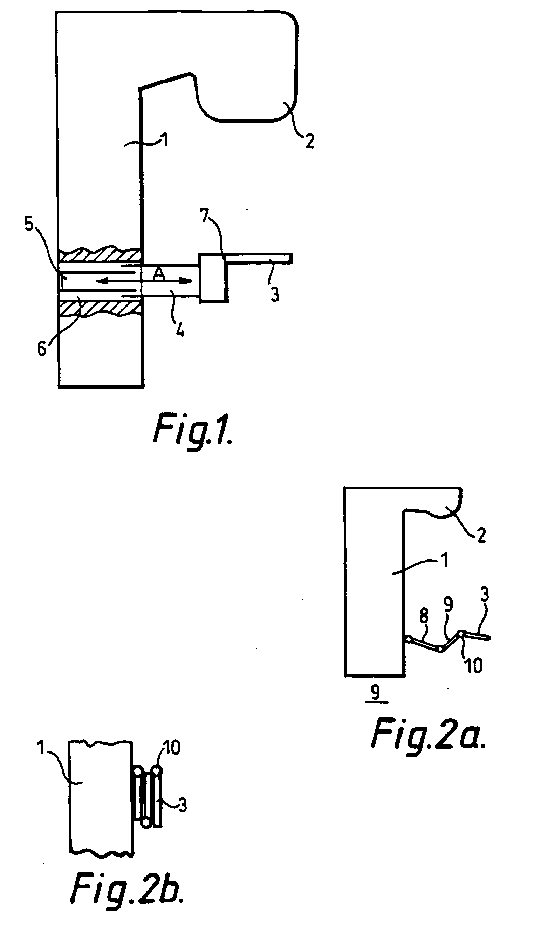

In Figure 1 a gantry 1 of a radiation therapy apparatus is provided with a radiation head 2 and diametrically opposed to the radiation head is an imaging device 3 connected to a telescopic arm 4, 5 which is retractable into a cavity 6 within the gantry 1. The arm comprises two concentrically aligned tubes 4, 5 slidable one within the other along an axis A. Imaging device 3 is pivotally mounted about a point 7 to the distal end of tube 4. The arrangement is shown in fully extended position, but it can be seen that retraction of tube 4 along tube 5 into cavity 6 and pivoting of imaging device 3 about pivot 7 allows the assembly to be retracted and stowed in a position flush with the surface of the gantry 1 of the radiation therapy apparatus.

In Figure 2 the gantry 1 of a radiation therapy apparatus has pivotally connected thereto a jointed arm 8, 9 which is further connected to an imaging device 3 about a pivotal joint 10. Figure 2a shows the arrangement partially extended and Figure 2b shows the arrangement fully retracted.

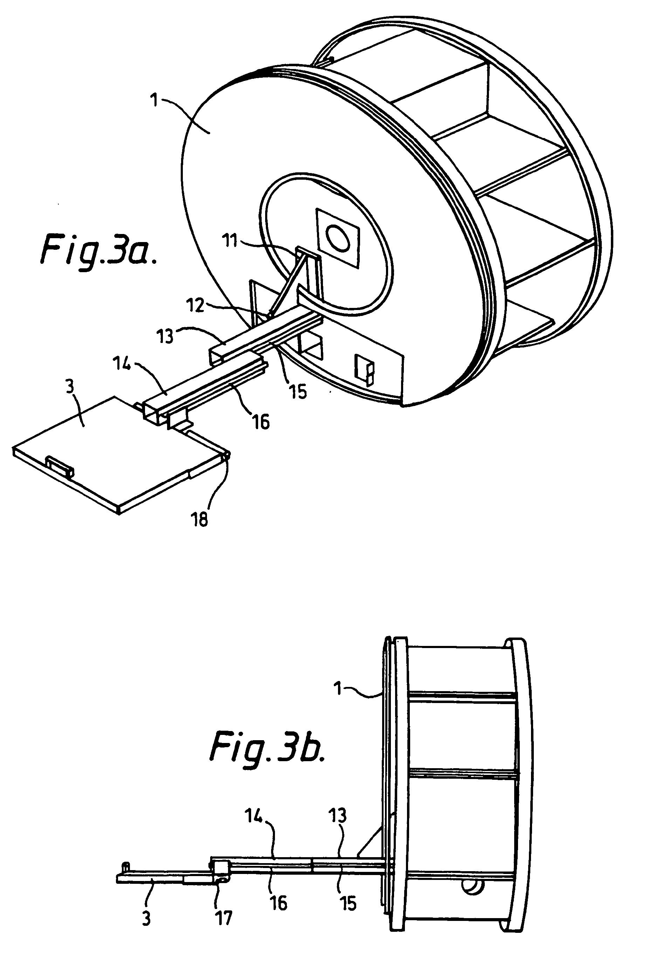

In Figure 3, a gantry 1 of a radiation therapy apparatus has fixed thereto a mounting plate 11 to which, by means of pivot 12, telescopically extending arm 13, 14 is attached. The telescopically extending arm comprises two tubes, 13 and 14, which in this embodiment are of substantially rectangular cross-section but may be of any other suitable cross-section arranged to slide side by side by means of linear bearing 15. Slidably connected to the distal portion 14 of the slidable arm is an imaging device holder 17 which is slidable along linear bearing 16. Mounted on the holder 17 is an imaging device 3. Imaging device 3 is slidable with respect to holder 17 by means of linear bearing 18.

As can be seen from the Figure the imaging device 3 is free to move along two perpendicular axes defined by linear bearings 16 and 18.

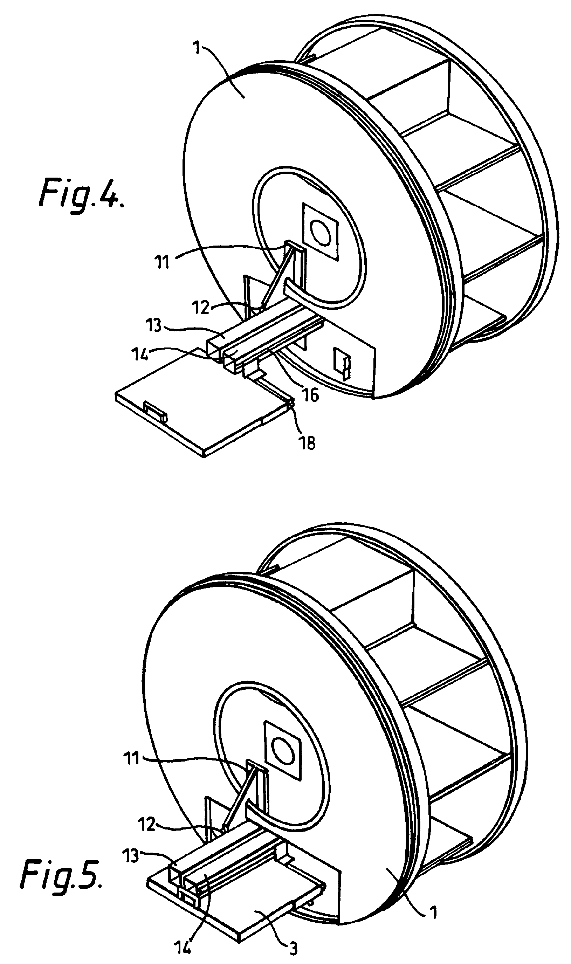

In Figure 4, the distal portion 14 of the extendable arm 13, 14 has been moved towards the surface of gantry 1 by means of linear bearing 15. Movement of the slidable arm 14 in linear bearing 15 together with movement of linear bearing 18 of holder 17 allows the imaging device 3 to be locatable about a relatively large viewing area.

In Figure 5 holder 17 has been moved from a relatively distal to a relatively proximal position on the extendable arm 13, 14 drawing the imaging device 3 closer to the surface of the gantry 1. This partially retracted position provides more convenient access to the patient during treatment.

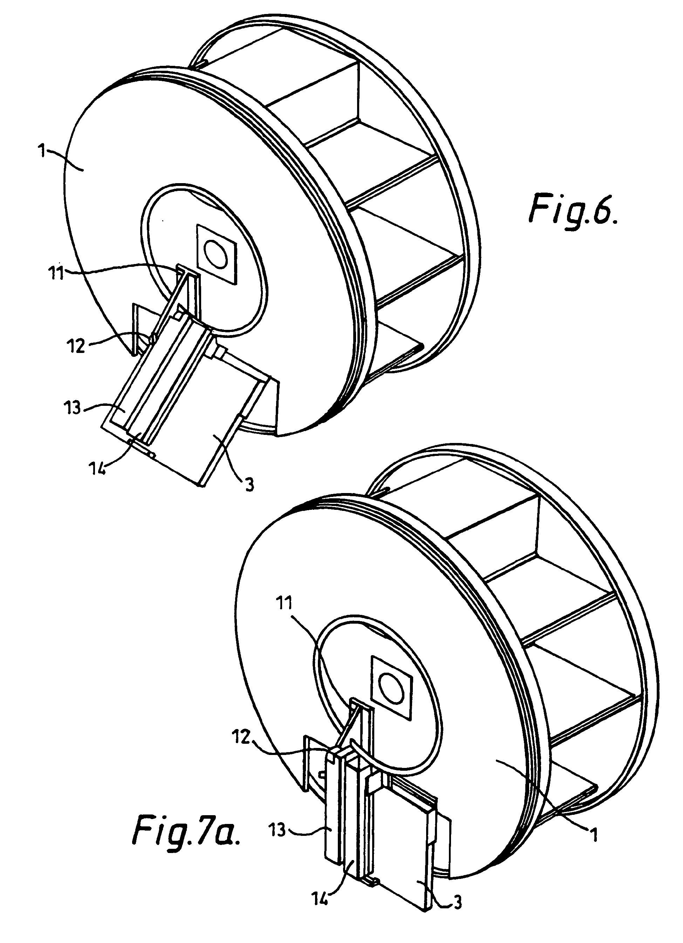

Figure 6 illustrates how the apparatus, fully retracted as shown in Figure 5, can be pivoted about pivot 12 towards a stowing position substantially flush with the surface of gantry 1.

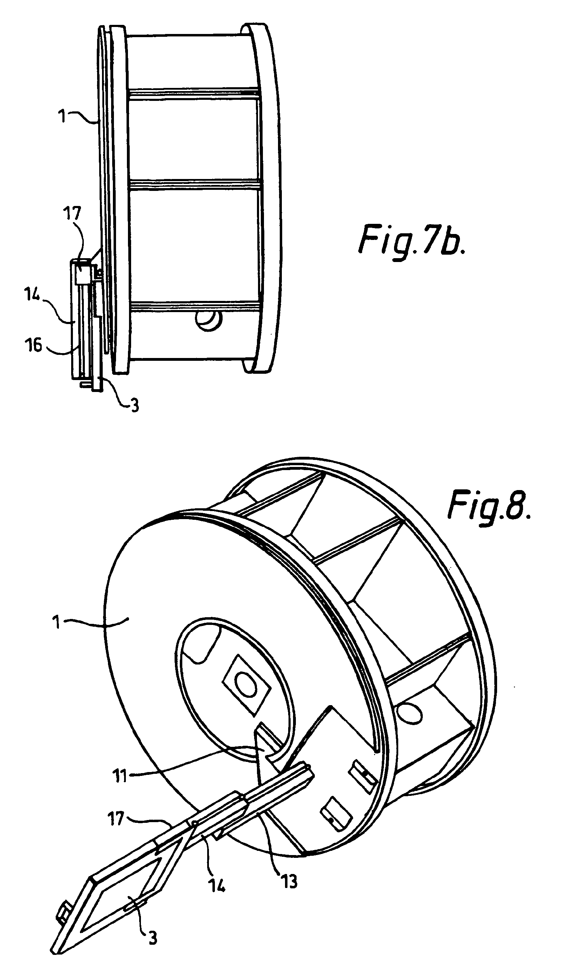

Finally, Figure 7 illustrates the apparatus in fully stowed position.

The foregoing describes only one embodiment of the invention to aid understanding and is not intended to be in any way limiting from the true scope of the invention as defined in the appended claims.

## Claims

1. Apparatus for positioning an imaging device relative to the gantry (1) of a radiation therapy apparatus comprises a mounting device (11) for mounting the apparatus on the gantry (1) surface, a telescopically extendable arm (13,14) pivotally (12) connected to the mounting device (11) and a holder (17) for holding an imaging device (3), the holder (17) being connected to the distal portion (14) of the telescopically extendable arm (13, 14) wherein the arm comprises two or more elongate elements (13, 14) in slidable communication with each other and the slidable communication is provided by one or more linear bearings (15) between the elongate elements (13, 14), **characterised in that** the holder (17) comprises means (18) for sliding the image device along an axis perpendicular to the longitudinal axis of the extendable arm (13, 14).

2. Apparatus as claimed in claim 1 **characterised in that** the elongate elements (13, 14) do not share a common central axis.

3. Apparatus as claimed in any preceding claim **characterised in that** the arm (13, 14) is pivotally mounted substantially about its centre of mass.

4. Apparatus as claimed in any one of claims 1 or 2 **characterised in that** the arm (13, 14) is pivotally mounted substantially about the centre of mass of the arm and imaging device (3) assembly.

5. Apparatus as claimed in any preceding claim **characterised in that** the holder (17) is slidably mounted to slide along the extendable arm (13, 14).

6. Apparatus as claimed in any preceding claim **characterised in that** the holder (17) is detachable from the imaging device (3) and/or the extendable arm (13, 14).

7. Apparatus as claimed in any preceding claim **characterised in that** the holder (17) comprises means for locking the position of the imaging device (3).

8. Apparatus as claimed in any preceding claim further comprising means for rotating the imaging device (3) about an axis parallel to longitudinal axis of the extendable arm (13, 14).

9. Apparatus as claimed in any preceding claim comprising a counterbalancing means for holding the extendable arm (13, 14) under gravity in any given angular position relative to the surface of the gantry (1).

10. Apparatus as claimed in any preceding claim **characterised in that** the apparatus is actuated by mechanical or electro-mechanical means.

11. A radiation therapy apparatus comprising a gantry (1) having mounted thereon apparatus for positioning an imaging device as described in any of claims 1 to 10.

12. A radiation therapy apparatus as claimed in claim 11 **characterised by** comprising a plurality of apparatus as claimed in any one of claims 1 to 10.

## Patentansprüche

1. Vorrichtung für das Positionieren einer Bildgebungseinrichtung in Bezug zur Gantry (1) einer Strahlentherapievorrichtung, die Folgendes umfasst: eine Befestigungseinrichtung (11) für das Befestigen der Vorrichtung an der Oberfläche der Gantry (1), einen teleskopartig ausfahrbaren Arm (13, 14) der schwenkbar (12) mit der Befestigungseinrichtung (11) verbunden ist, und einen Halter (17) für das Halten einer Bildgebungseinrichtung (3), der mit dem distalen Abschnitt (14) des teleskopartig ausfahrbaren Arms (13, 14) verbunden ist, wobei der Arm zwei oder mehrere längliche Elemente (13, 14) umfasst, die verschiebbar miteinander verbunden sind, und die verschiebbare Verbindung durch ein oder mehrere Linearlager (15) zwischen den länglichen Elementen (13, 14) bereitgestellt wird, **dadurch gekennzeichnet, dass** der Halter (17) ein Mittel (18) umfasst, mit dem die Bildgebungseinrichtung an einer Achse entlang verschoben werden kann, die senkrecht zur Längsachse des ausfahrbaren Arms (13, 14) verläuft.

2. Vorrichtung nach Anspruch 1, **dadurch gekennzeichnet, dass** die länglichen Elemente (13, 14) nicht die gleiche Mittelachse besitzen.

3. Vorrichtung nach einem der vorhergehenden Ansprüche, **dadurch gekennzeichnet, dass** der Arm (13, 14) im Wesentlichen um seinen Schwerpunkt verschwenkbar angebracht ist.

4. Vorrichtung nach einem der Ansprüche 1 und 2, **dadurch gekennzeichnet, dass** der Arm (13, 14) im Wesentlichen um den Schwerpunkt der Baugruppe aus Arm und Bildgebungseinrichtung (3) verschwenkbar angebracht ist.

5. Vorrichtung nach einem der vorhergehenden Ansprüche, **dadurch gekennzeichnet, dass** der Halter (17) so verschiebbar angebracht ist, dass er an dem ausfahrbaren Arm (13, 14) entlang verschoben wird.

6. Vorrichtung nach einem der vorhergehenden Ansprüche, **dadurch gekennzeichnet, dass** der Halter (17) von der Bildgebungseinrichtung (3) und/ oder dem ausfahrbaren Arm (13, 14) abgenommen werden kann.

7. Vorrichtung nach einem der vorhergehenden Ansprüche, **dadurch gekennzeichnet, dass** der Halter (17) ein Mittel umfasst, mit dem die Position der Bildgebungseinrichtung (3) fixiert werden kann.

8. Vorrichtung nach einem der vorhergehenden Ansprüche, die des Weiteren ein Mittel umfasst, mit dem die Bildgebungseinrichtung (3) um eine Achse gedreht werden kann, die parallel zur Längsachse des ausfahrbaren Arms (13, 14) verläuft.

9. Vorrichtung nach einem der vorhergehenden Ansprüche, die ein Ausgleichsmittel umfasst, mit dem der ausfahrbare Arm (13, 14) unter Schwerkraft in jeder gegebenen Winkelposition zur Oberfläche der Gantry (1) gehalten werden kann.

10. Vorrichtung nach einem der vorhergehenden Ansprüche, **dadurch gekennzeichnet, dass** sie durch ein mechanisches oder elektromechanisches Mittel betätigt wird.

11. Strahlentherapievorrichtung, die eine Gantry (1) umfasst, an der eine in einem der Ansprüche 1 bis 10 beschriebene Vorrichtung für das Positionieren einer Bildgebungseinrichtung befestigt ist.

12. Strahlentherapievorrichtung nach Anspruch 11, **dadurch gekennzeichnet, dass** sie mehrere Vorrichtungen nach einem der Ansprüche 1 bis 10 umfasst.

## Revendications

1. Appareil de positionnement d'un dispositif d'imagerie relativement au statif (1) d'un appareil de radiothérapie, comprenant un dispositif de montage (11) pour monter l'appareil sur la surface du statif (1), un bras télescopique extensible (13,14) connecté de façon pivotante (12) au dispositif de montage (11) et un élément de maintien (17), pour maintenir un dispositif d'imagerie (3), le dispositif de maintien (17) étant connecté à la partie distale (14) du bras télescopique extensible (13,14), dans lequel le bras comprend deux éléments allongés (13,14) ou davantage, en communication mutuelle coulissante, la communication coulissante étant assurée par un palier lisse (15), ou davantage, placé entre les éléments allongés (13,14), **caractérisé en ce que** le dispositif de maintien (17) comprend des moyens (18) pour faire coulisser le dispositif d'imagerie le long d'un axe perpendiculaire à l'axe longitudinal du bras extensible (13,14).

2. Appareil selon la revendication 1, **caractérisé en ce que** les éléments allongés (13,14) ne se partagent pas un axe central commun.

3. Appareil selon l'une quelconque des revendications précédentes, **caractérisé en ce que** le bras (13,14) est monté de façon pivotante de sorte à pivoter sensiblement autour de son centre d'inertie.

4. Appareil selon l'une quelconque des revendications 1 ou 2, **caractérisé en ce que** le bras (13,14) est monté de sorte à pivoter sensiblement autour de l'ensemble formé par le bras et le dispositif d'imagerie (3).

5. Appareil selon l'une quelconque des revendications précédentes, **caractérisé en ce que** le dispositif de maintien (17) est monté de façon coulissante pour coulisser le long du bras extensible (13,14).

6. Appareil selon l'une quelconque des revendications précédentes, **caractérisé en ce que** le dispositif de maintien (17) peut être détaché du dispositif d'imagerie (3) et/ou du bras extensible (13,14).

7. Appareil selon l'une quelconque des revendications précédentes, **caractérisé en ce que** le dispositif de maintien (17) comprend des moyens de verrouillage en position du dispositif d'imagerie (3).

8. Appareil selon l'une quelconque des revendications précédentes, qui comprend de plus des moyens de rotation du dispositif d'imagerie (3) autour d'un axe parallèle à l'axe longitudinal du bras extensible (13,14).

9. Appareil selon l'une quelconque des revendications précédentes, qui comprend des moyens formant contrepoids pour maintenir sous gravité le bras extensible (13,14) en n'importe quelle position angulaire relativement à la surface du statif (1).

10. Appareil selon l'une quelconque des revendications précédentes, **caractérisé en ce que** l'appareil est actionné par des moyens mécaniques ou électromécaniques.

11. Appareil de radiothérapie comprenant un statif (1) sur lequel est monté un appareil pour le positionnement d'un dispositif d'imagerie tel que décrit dans l'une quelconque des revendications 1 à 10.

12. Appareil de radiothérapie selon la revendication 11, **caractérisé en ce qu'**il comprend une pluralité d'appareils tels que revendiqués dans l'une quelconque des revendications 1 à 10.
